# EUROPÄISCHE PATENTSCHRIFT

(11) **EP 1 308 504 B1**
(45) Veröffentlichungstag und Bekanntmachung des Hinweises auf die Patenterteilung: **17.01.2007**
(21) Anmeldenummer: 02021917.6
(22) Anmeldetag: 27.09.2002
(51) Int. Cl.: C12M 1/00

(54) **Inkubations-und Lagervorrichtung, insbesondere für Proben aus organischem Material**
Apparatus for the incubation and storage especially for samples of organic material
Appareil pour l'incubation et le stockage, spécialement pour des échantillons de matière organique

(30) Priorität: 01.11.2001 DE 10154663
(43) Veröffentlichungstag der Anmeldung: 07.05.2003
(73) Patentinhaber: Thermo Electron LED GmbH, 63505 Langenselbold (DE)
(72) Erfinder: Kauschke, Marion Dr., 63739 Aschaffenburg (DE); Bidlingmaier, Dieter, 63486 Bruchköbel (DE)
(74) Vertreter: Lang, Friedrich

(56) Entgegenhaltungen:
- EP-A- 0 967 268
- EP-A- 1 148 120
- WO-A-97/21834
- DE-A- 19 925 154
- DE-A- 19 952 330
- DE-U- 9 403 392
- US-A- 2 124 250

## Beschreibung

Die Erfindung betrifft eine Inkubations- und Lagervorrichtung, insbesondere für Proben aus organischem Material.

Vorrichtungen der genannten Art werden in Biologie und Medizin beispielsweise dazu verwendet, um organische Materialien unter definierten äußeren Bedingungen zu lagern.

Große Verbreitungen haben derartige Vorrichtungen für die Aufzucht und Lagerung von Mikroorganismen, welche unter speziellen Lebensbedingungen ihr Wachstum stark beschleunigen können. Im Allgemeinen wird in diesen Vorrichtungen die Temperatur als wichtiger Wachstumsparameter auf einem bestimmten Niveau für eine längere Zeit konstant gehalten. Es sind aber auch Anwendungsfälle bekannt, bei denen nach einem vorgegebenen Temperaturregime in bestimmten Zeiten bestimmte Temperaturen erreicht und für eine gewisse Zeit konstant gehalten werden. Dabei sind diese Geräte häufig sowohl für einen Heiz- als auch für einen Kühlbetrieb mit Heizvorrichtungen und Kälteanlagen ausgestattet.

In der Medizin finden temperierbare Inkubationsvorrichtungen oder Lagereinrichtungen zur Lagerung von Geweben Einsatz.

Dabei ist es sehr wichtig, dass diese Vorrichtungen aus hygienischen Gründen sehr leicht zu reinigen sind. Insbesondere ist dies erforderlich, um eine Keimverschleppung und unerwünschte Infektionen zu verhindern.

Im Stand der Technik sind verschiedene Grundformen von Inkubations- und Lagervorrichtungen bekannt.

Man unterscheidet zwischen folgenden Systemen:

### 1. Direkt-Verdampfer für Kältemittel und elektrischen Heizelementen

Dabei sind die elektrischen Heizelemente von außen auf dem Innenbehälter der Inkubationsvorrichtung aufgebracht oder auch in seltenen Fällen im Innenraum angeordnet. Die Kühlung erfolgt über die Kältemittelverdampfer, die beispielsweise direkt im Innenraum angeordnet sind. Alternativ wird ein Teil der Innenraumluft über einen Bypass zum Verdampfer geführt und gekühlt, bevor die abgekühlte Luft wieder dem Innenraum der Inkubationsvorrichtung zugeführt wird.

Als Nachteil dieser bekannten Lösung ist hervorzuheben, dass die beiden voneinander getrennten Systeme der Beheizung und Kühlung der Inkubationsvorrichtung nur sehr aufwendig und schwierig in Bezug auf die Einhaltung der vorgegebenen Temperatur zu regeln sind. Die Überlagerung von Kühlung und Heizung ist infolge der Trägheit der Einzelsysteme häufig sehr groß, so dass entweder Abstriche an die Temperaturkonstanz gemacht werden müssen oder aber aufwändige Regelsysteme zu installieren sind. Insbesondere schwierig ist es, das Heiz- und Kühlsystem so aufeinander abzustimmen, dass eine Temperaturkonstanz mit geringen Abweichungen der Temperatur um einen Sollwert erreicht wird.

### 2. Indirekte Kühlung und elektrische Heizelemente

Ein solches System wird in der DE 873892 offenbart. Dabei sind im Innenraum rohrförmige Heizelemente in Trägern angeordnet, welche nach einer Ausgestaltung des Gegenstandes der genannten Erfindung auch teilweise durch Kühlrohre ersetzt werden können. Dadurch wird die kombinierte Kühlung und Beheizung des Innenraumes ermöglicht.

Wiederum ist als Nachteil dieses Standes der Technik die aufwändige Regelungstechnik zu nennen, um die voneinander getrennten Systeme der Heizung und Kühlung des Innenraumes der Inkubationsvorrichtung aufeinander abzustimmen. Darüber hinaus sind die Träger, in denen sich die Heizelemente oder die Kühlflüssigkeit befinden, im Innenraum angeordnet, was die Reinigungsmöglichkeiten bei hygienisch anspruchsvollen Anwendungen in der Biologie und der Medizin stark beeinträchtigt.

### 3. Wassermantel

Bei diesem System wird als Wärmeträger Wasser verwendet, welches im sogenannten "Wassermantel" zirkuliert. Der Wassermantel ist dabei als zweiter Behälter um den Innenbehälter herum angeordnet. Dadurch ist die Temperaturkonstanz zwar sehr gut, aber das System ist außerordentlich träge. Temperaturregime mit kurzen Phasen verschiedener Temperatur sind mit diesem System praktisch nicht realisierbar. Zudem ist weiterhin von Nachteil, dass dieses System nicht ohne weiteres gekühlt werden kann, da ein Verdampfer binnen kurzer Zeit vereist. Aus diesem Grund sind diese Systeme nur für Temperaturen oberhalb der Raumtemperatur geeignet und ohne Kühlsystem erhältlich.

Als weiterer Nachteil der direkten Beheizung ist zu nennen, dass die verwendeten Elektroheizelemente bei einer relativ geringen Oberfläche eine sehr hohe Temperatur erzeugen, wobei die Wärme durch Strahlung und Konvektion an die Luft im Innenraum der Inkubationsvorrichtung abgegeben wird. Da dort aber nur beispielsweise 37°C gefordert sind, entstehen Strömungen unterschiedlicher Temperatur, ehe sich der gewünschte Mittelwert einstellt. Es ist somit nicht nur regelungstechnisch, sondern auch strömungstechnisch schwierig, die Temperatur im Innenraum gleichmäßig zu verteilen.

Es ist Aufgabe der Erfindung, eine Inkubations- und Lagervorrichtung derart auszubilden, dass diese zur Temperierung von Proben aus organischem Material geeignet ist und darüber hinaus im Innenraum leicht zu reinigen ist. Zudem soll die konstante Einhaltung der Temperatur im Innenraum sichergestellt werden.

Erfindungsgemäß wird die Aufgabe dadurch gelöst, dass die Inkubations- und Lagervorrichtung indirekt über einen flüssigen Wärmeträger beheizt und gekühlt wird. Dazu stehen die Begrenzungsflächen des Innenraumes der Inkubations- und Lagervorrichtung mit von außen angebrachten Wärmeübertragerelementen thermisch in Verbindung, wobei die Wärmeüber tragerelemente wiederum von einem flüssigen Wärmeträger im Kreislauf durchströmt werden. Ebenso sind Mittel zur Kühlung und Erwärmung des Wärmeträgers erfindungsgemäß vorgesehen.

Durch den Einsatz eines Wärmeträgers ist die erforderliche Temperaturkonstanz vorteilhaft im Inneren der Vorrichtung gut zu erreichen.

Besonders bei Innenraumtemperaturen nahe der Umgebungstemperatur macht sich die kombinierte indirekte Kühlung und Beheizung vorteilhaft bemerkbar.Eine dynamische Temperaturführung des Innenraums wird über das System, der an einen Wärmeträgerkreislauf gekoppelten Erwärmung und Kühlung, möglich, wodurch eine Temperaturänderung aufgrund veränderter eingebrachter Leistungen vermieden werden kann.

Weitere Einzelheiten, Merkmale und Vorteile der Erfindung ergeben sich aus der nachfolgenden Beschreibung von Ausführungsbeispielen mit Bezugnahme auf die zugehörige Zeichnung. Es zeigt:

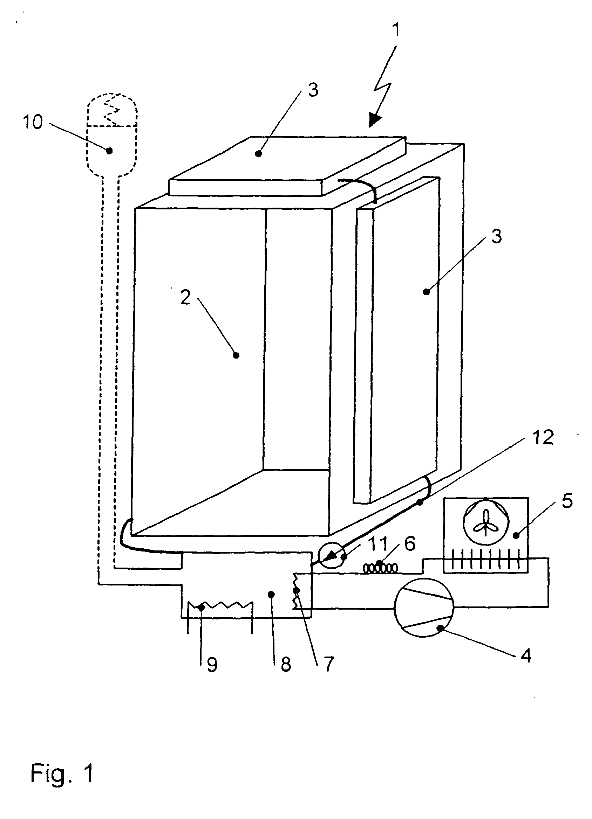

Fig. 1: perspektivische Prinzipdarstellung einer Inkubations- und Lagervorrichtung

In Fig.1 ist eine erfindungsgemäße Ausgestaltung einer Inkubations- und Lagervorrichtung perspektivisch dargestellt. Der Innenraum 2 wird durch innere Begrenzungsflächen gebildet, auf denen Wärmeübertragerelemente 3 von außen angeordnet sind.

Die Wärmeübertragerelemente 3 stehen thermisch mit den Begrenzungsflächen des Innenraumes 2 in Verbindung oder bilden selbst einen Teil der Begrenzungsflächen.

Ein flüssiger Wärmeträger durchströmt im Kreislauf die 10 Wärmeübertragerelemente 3 und nimmt dabei, je nach Temperatur, Wärme aus dem Innenraum 2 der Inkubations- und Lagervorrichtung 1 auf oder gibt Wärme an den Innenraum 2 ab. Der flüssige Wärmeträger wird mittels einer Pumpe 11 bevorzugt im Kreislauf gepumpt.

Die Wärmeübertragerelemente 3 sind gemäß der dargestellt bevorzugten Ausführungsform der Erfindung kastenförmig ausgebildet. Diese kastenförmigen Wärmeübertragerelemente 3 an den Begrenzungsflächen des Innenraumes 2 sind durch Rohrverbindungen 12 miteinander verbun-

Die Wärmeträgerflüssigkeit wird durch einen Wärmeübertrager 9 einer nicht dargestellten Heizung erwärmt oder aber durch den Wärmeübertrager/Verdampfer 7 einer Kälteanlage gekühlt.

Die Kälteanlage weist dazu einen Verdichter 4 auf, in welchem das dampfförmige Kältemittel verdichtet wird. Im Kondensator 5, im Ausführungsbeispiel als Luftverflüssiger dargestellt, wird das Kältemittel verflüssigt, im Entspannungsorgan 6 entspannt und gelangt schließlich in den Verdampfer 7, wo es unter Wärmeaufnahme vom Wärmeträger verdampft. Der Kältemitteldampf wird vom Verdichter 4 angesaugt, womit sich der Kältemittelkreislauf schließt.

Das Wärmeträgersystem wird alternativ vorteilhaft vervollständigt durch einen Ausgleichsbehälter 10, der die durch Temperaturwechsel verursachten Volumenschwankungen des Wärmeträgers ausgleicht. Die beiden Wärmeübertrager 7, 9 zur Kühlung und Heizung des Wärmeträgers werden gemäß der bevorzugten Ausführungsform in einem Wärmeträgerreservoir 8 angeordnet. Hierdurch ergibt sich für die Regelung des Wärmeträgerkreislaufes eine vorteilhafte Dynamik.

Die Wärmeübertragerelemente 3 sind nach einer weiteren Ausführungsform der Erfindung als Rohrschlangen ausgebildet. Diese verlaufen um die 10 Begrenzungsflächen des Innenraumes 2 herum und sind mit diesen thermisch verbunden.

Ebenso vorteilhaft ist es, zur Temperierung der Inkubations- und Lagervorrichtung einen externen Thermostaten mit Kühl- und Heizfunktion oder ein internes Heizgerät und optional ein externes Kühlgerät zu verwenden. Dazu sind die externen Komponenten mit den erforderlichen Anschlüssen und einem Behälter für die Aufnahme des Wärmeträgers und einer Pumpe auszustatten. Des Weiteren sind die externen Komponenten mit einer eigenen Temperaturregelung versehen.

Bei einer Inkubations- und Lagervorrichtung mit einer externen Kälteanlage wird das Wärmeträgersystem vorteilhaft als offenes bzw. erweiterbares System ausgeführt. Somit können Kältekapazitätsreserven der Kälteanlage über vorzusehende Anschlüsse beim Wärmeträgersystem für andere Anwendungen genutzt werden.

Um die Kondensatbildung von Luftfeuchtigkeit an Sichtfenstern der Inkubations- und Lagervorrichtung zu verhindern, werden die transparenten Flächen, wie beispielsweise Glasscheiben, beheizbar ausgeführt.

Die Erwärmung des Wärmeträgers der Inkubations- und Lagervorrichtung wird vorteilhaft durch eine Widerstandsheizung vorgenommen.
Als Kältemittel der Kälteanlage kommt bevorzugt R134a, Propan, Butan oder Kohlendioxid zum Einsatz.

Ein Gemisch aus Wasser und Glykol wird bevorzugt als Wärmeträger für die erfindungsgemäße Inkubations- und Lagervorrichtung eingesetzt.

### LISTE DER BEZUGSZEICHEN

- 1: Inkubations- und Lagervorrichtung
- 2: Innenraum
- 3: Wärmeübertragerelement
- 4: Verdichter
- 5: Kondensator
- 6: Entspannungsorgan
- 7: Wärmeübertrager/Verdampfer
- 8: Wärmeträgerreservoire
- 9: Wärmeübertrager Heizung
- 10: Ausgleichsbehälter Wärmeträgersystem
- 11: Pumpe
- 12: Rohrverbindung

## Patentansprüche

1. Inkubations- und Lagervorrichtung, insbesondere für Proben aus organischem Material, **dadurch gekennzeichnet,**
**dass** die Begrenzungsflächen des Innenraumes (2) der Inkubations- und Lagervorrichtung (1) mit von außen angebrachten Wärmeübertragerelementen (3) thermisch in Verbindung stehen und dass die Wärmeübertragerelemente (3) von einem flüssigen Wärmeträger im Kreislauf durchströmt werden, wobei Mittel zur Kühlung und Erwärmung des Wärmeträgers vorgesehen sind und dass die Temperierung des Innenraumes (2) indirekt über den flüssigen Wärmeträger erfolgt.

2. Inkubations- und Lagervorrichtung nach Anspruch 1, **dadurch gekennzeichnet, dass** die Wärmeübertragerelemente (3) kastenförmig ausgebildet sind.

3. Inkubations- und Lagervorrichtung nach Anspruch 1, **dadurch gekennzeichnet, dass** die Wärmeübertragerelemente (3) als Rohrschlangen ausgebildet sind.

4. Inkubations- und Lagervorrichtung nach einem der Ansprüche 1 bis 3, **dadurch gekenn-zeichnet, dass** für den Kreislauf des Wärmeträgers die Wärmeübertragerelemente (3) und eine Pumpe (11) durch Rohrverbindungen (12) miteinander verbunden sind und dass weiterhin ein Wärmeübertrager (9) für die Erwärmung und ein Wärmeübertrager/Verdampfer (7) für die Kühlung des Wärmeträgers vorgesehen sind.

5. Inkubations- und Lagervorrichtung nach einem der Ansprüche 1 bis 4, **dadurch gekennzeichnet, dass** im Kreislauf für den Wärmeträger ein Wärmeträgerreservoire (8) vorgesehen ist und dass der Wärmeübertrager (9) und der Verdampfer (7) im Wärmeträgerreservoire (8) angeordnet sind.

6. Inkubations- und Lagervorrichtung nach einem der Ansprüche 1 bis 5, **dadurch gekennzeichnet, dass** im Kreislauf für den Wärmeträger ein Ausgleichsbehälter (10) vorgesehen ist.

7. Inkubations- und Lagervorrichtung nach einem der Ansprüche 1 bis 6, **dadurch gekennzeichnet, dass** zur Erwärmung des Wärmeträgers eine Widerstandsheizung vorgesehen ist.

8. Inkubations- und Lagervorrichtung nach einem der Ansprüche 1 bis 7, **dadurch gekennzeichnet, dass** zur Kühlung des Wärmeträgers ein Kältemittelkreislauf vorgesehen ist.

## Claims

1. An incubation and storage apparatus, especially for samples of organic material, **characterized in that** the delimiting surfaces of the inside space (2) of the incubation and storage apparatus (1) are in thermal connection with heat-transfer elements (3) attached from the outside and that the heat-transfer elements (3) are flowed through by a liquid heat-transfer medium in the circulation, with means being provided for cooling and heating the heat-transfer medium and that the tempering of the inside space (2) occurs indirectly via the liquid heat-transfer medium.

2. An incubation and storage apparatus according to claim 1, **characterized in that** the heat-transfer elements (3) are provided with a box-type configuration.

3. An incubation and storage apparatus according to claim 1, **characterized in that** the heat-transfer elements (3) are arranged as tube coils.

4. an incubation and storage apparatus according to one of the claims 1 to 3, **characterized in that** the heat-transfer elements (3) and a pump are mutually connected by means of pipe connections (12) for the circulation of the heat-transfer medium and that a heat exchanger (9) is provided for heating and a heat exchanger/evaporator (7) is provided for cooling the heat-transfer medium.

5. An incubation and storage apparatus according to one of the claims 1 to 4, **characterized in that** a reservoir (8) for the heat-transfer medium is provided in the circulation and that the heat exchanger (9) and the evaporator (7) are arranged in the reservoir (8) for the heat-transfer medium.

6. An incubation and storage apparatus according to one of the claims 1 to 5, **characterized in that** a compensating reservoir (10) is provided in the circulation for the heat-transfer medium.

7. An incubation and storage apparatus according to one of the claims 1 to 6, **characterized in that** a resistance heating is provided for heating the heat-transfer medium.

8. An incubation and storage apparatus according to one of the claims 1 to 7, **characterized in that** a coolant circulation is provided for cooling the heat-transfer medium.

## Revendications

1. Dispositif d'incubation et de stockage, en particulier pour des échantillons de matières organiques, **caractérisé en ce que** les surfaces délimitant l'espace intérieur (2) du dispositif d'incubation et de stockage (1) sont en liaison thermique avec des éléments caloporteurs (3) appliqués par l'extérieur et **en ce que** les éléments caloporteurs (3) sont parcourus par un liquide caloporteur en circuit, des moyens étant prévus pour refroidir et chauffer le caloporteur, et **en ce que** la mise à température de l'espace intérieur (2) est réalisée indirectement à l'aide du liquide caloporteur.

2. Dispositif d'incubation et de stockage selon la revendication 1, **caractérisé en ce que** les éléments caloporteurs (3) sont conçus en forme de caissons.

3. Dispositif d'incubation et de stockage selon la revendication 1, **caractérisé en ce que** les éléments caloporteurs (3) sont conçus comme des serpentins de tuyau.

4. Dispositif d'incubation et de stockage selon l'une des revendications 1 à 3, **caractérisé en ce que** pour le circuit du caloporteur, les éléments caloporteurs (3) sont mis en communication avec une pompe (11) par des raccords de tube (12) et **en ce qu'**il est en outre prévu un échangeur de chaleur (9) pour chauffer le caloporteur et un échangeur de chaleur/évaporateur (7) pour le refroidir.

5. Dispositif d'incubation et de stockage selon l'une des revendications 1 à 4, **caractérisé en ce qu'**il est prévu dans le circuit de caloporteur un réservoir de caloporteur (8) et **en ce que** l'échangeur de chaleur (9) et l'évaporateur (7) sont disposés dans le réservoir de caloporteur (8).

6. Dispositif d'incubation et de stockage selon l'une des revendications 1 à 5, **caractérisé en ce qu'**un réservoir d'équilibrage (10) est prévu dans le circuit de caloporteur.

7. Dispositif d'incubation et de stockage selon l'une des revendications 1 à 6, **caractérisé en ce qu'**il est prévu un chauffage à résistance pour le chauffage du caloporteur.

8. Dispositif d'incubation et de stockage selon l'une des revendications 1 à 7, **caractérisé en ce qu'**il est prévu un circuit de réfrigérant pour le refroidissement du caloporteur.
